# FASCICULE DE BREVET EUROPEEN

(11) **EP 0 851 855 B1**
(45) Date de publication et mention de la délivrance du brevet: **05.06.2002**
(21) Numéro de dépôt: 96928490.0
(22) Date de dépôt: 07.08.1996
(51) Int. Cl.: C07D 209/16, A61K 31/40, C07D 209/34, C07D 401/12, C07C 233/31

(54) **DERIVES ACYLES DE LA MELATONINE ET D'ANALOGUES EN TANT QUE MEDICAMENT**
ACYLIERTE DERIVATE VON MELATONIN UND DESSEN ANALOGE ALS ARZNEIMITTEL
ACYLATED DERIVATIVES OF MELATONIN AND ITS ANALOGUES, USEFUL AS MEDICAMENTS

(30) Priorité: 08.08.1995 FR 9509611
(43) Date de publication de la demande: 08.07.1998
(73) Titulaire: MACEF, 33000 Bordeaux (FR); LABORATOIRES BESINS INTERNATIONAL, 75003 Paris (FR)
(72) Inventeur: FOURTILLAN, Jean-Bernard, F-86440 Migne-Auxances (FR); FOURTILLAN, Marianne, F-86440 Migne-Auxances (FR); JACQUESY, Jean-Claude, F-86180 Buxerolles (FR); JOUANNETAUD, Marie-Paule, F-86000 Poitiers (FR); VIOLEAU, Bruno, F-86370 Marcay (FR); KARAM, Omar, F-86000 Poitiers (FR)
(74) Mandataire: Warcoin, Jacques
(86) Numéro de dépôt international: FR9601260
(87) Numéro de publication internationale: WO9706140

(56) Documents cités:
- EP-A- 0 527 687
- EP-A- 0 585 206
- US-A- 5 403 851

## Description

La présente invention concerne des dérivés agonistes mélatoninergiques, leur procédé de préparation et leur utilisation à titre de médicament.

La mélatonine, N-acétyl-5-méthoxytryptamine, est une hormone de la glande pinéale, isolée par Lerner *et al*. (J.am.Chem.Soc., 80, 1958, 2587), qui a fait l'objet de nombreuses études pour son activité circadienne, dans le rythme du sommeil, pour ses effets sur la production de testostérone, pour son activité au niveau de l'hypothalamus et dans les désordres psychiatriques.

Il a ainsi été envisagé d'employer la mélatonine et ses analogues, notamment pour le traitement de la dépression de et des désordres psychiatriques, en particulier le stress, l'anxiété, la dépression, l'insomnie, la schizophrénie, les psychoses, l'épilepsie, mais également pour le traitement des troubles du sommeil liés aux voyages (« jet lag »), des maladies neurodégénératives du système nerveux central comme la maladie de Parkinson ou la maladie d'Alzheimer, pour le traitement de cancers, ou encore comme contraceptif, ou comme analgésique.

Toutefois, l'utilisation directe de la mélatonine in vivo ne s'est pas montrée très satisfaisante, compte tenu d'un premier passage hépatique qui extrait plus de 90% du principe actif, administré par voie orale.

Différents analogues de la mélatonine ont été décrits, mettant en évidence deux voies de recherche qui portent soit sur les substituants de la mélatonine (WO-A-89/01472, US-A-5 283 343, US-A-5 093 352 ou WO-A-93/11761) soit sur le noyau aromatique en remplaçant le groupe indolyle par un naphtyle (FR-A-2 658 818, FR-A 2 689 124).

US 5 403 851, EP 527 687 et EP 585 206 décrivent des composés de formules générales apparentées à celle des composés de la présente demande ainsi que l'utilisation de ces composés pour le traitement des troubles du rythme circadien et du sommeil et les désordres saisonniers.

La présente demande de brevet propose une nouvelle voie de développement d'analogues de la mélatonine présentant une activité améliorée.

L'étude de la technique antérieure a mis en évidence trois documents qui illustrent le domaine de l'invention : EP 527 687 qui concerne des dérivés d'aryléthylamines possédant un caractère agoniste ou antagoniste de la mélatonine. EP 585 206 et US 5 403 851 concernent des dérivés de tryptamines et phénalkylamines pour le traitement de pathologies liées à des troubles du rythme circadien.

Aucun de ces documents ne concerne des composés diacylés.

La présente invention concerne un dérivé choisi dans le groupe constitué par :
- les dérivés de formule générale I 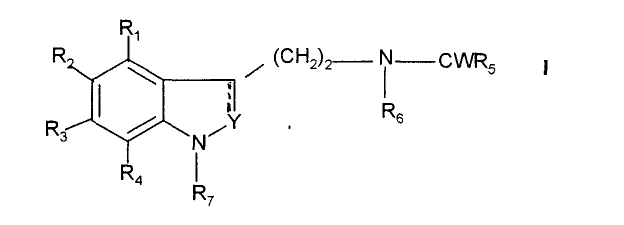
dans laquelle
**W** représente un atome d'oxygène ou de soufre ou un radical =NR₁₂, R₁₂ étant un atome d'hydrogène, un radical alkyle en C₁-C₆, aryle, aralkyle dont le reste alkyle est en C₁-C₆, cycloalkyle en C₃-C₆,
**Y ---** représente un radical de formule CR₈ =, CW₁ - ou CHR₂₀ -, W₁ ayant la même définition que W ci-dessus,
**R**_{**1**} **à R**_{**4**} représentent indépendamment l'un de l'autre un atome d'hydrogène, un radical hydroxyle, un radical alkyle en C₁-C₆, alkoxy dont le reste alkyle est en C₁-C₆, aryloxy, aralkoxy dont le reste alkyle est en C₁-C₆, halogéno ou nitro,
**R**_{**5**} représente un atome d'hydrogène, un radical alkyle en C₁-C₆ ,aryle, aralkyle dont le reste alkyle est en C₁-C₆, un radical perhalogéno-alkyle en C₁-C₆, un radical amino, alkyl-(en C₁-C₆)-amino ou dialkyl-( en C₁-C₆)-amino, ou un radical alkoxy dont le reste alkyle est en C₁-C₆.
**R**_{**6**} **et R**_{**20**} représentent indépendamment l'un de l'autre un atome d'hydrogène, un radical hydroxyle, un radical alkyle en C₁-C₆, cycloalkyle en C₃-C₆, alkoxy dont le reste alkyle est en C₁-C₆, aryloxy, aralkoxy dont le reste alkyle est en C₁-C₆, alkyl-(en C₁-C₆) thio, arylthio, aralkyl-(en C₁-C₆)-thio, halogéno, nitro ou une chaîne aliphatique insaturée, alkenyle en C₂-C₆, alkynyle en C₂-C₆, alkyle en C₁-C₆, aryle, aralkyle dont le reste alkyle est en C₁-C₆, un radical perhalogéno-alkyle en C₁-C₆, un radical amino, alkyl-(en C₁-C₆)-amino, dialkyl-(en C₁-C₆)-amino, arylamino, diarylamino, aralkylamino dont le reste alkyle est en C₁-C₆, un radical de la forme CV - R₁₁ ou QCVR₁₁, dans lequel V représente un atome d'oxygène ou de soufre ou un radical imine = N - R₁₂, R₁₁ a l'une des significations de R₁,
dans lesquels le terme aryle représente un groupe phényle, thiényle, furanyle, pyridile ou naphtyle, éventuellement substitué par un ou plusieurs substituants choisis parmi les radicaux alkyles en C₁-C₆, alkoxy dont le reste alkyle est en C₁-C₆ ou halogèno,
**Q** représente un atome d'oxygène ou de soufre.
**R**_{**7**} a l'une des significations de R₁ sauf qu'il ne peut être hydroxyle, ou il peut représenter un radical SO₂R₂₆, R₂₆ étant un radical alkyle en C₁-C₆ ou haloalkyle en C₁-C₆, notamment CF₃,
**R**_{**8**} a l'une des significations de R₁ et peut également représenter un atome d'halogène (chlore, brome, iode, fluor), un groupe Q'-CV'-R'₁₁ dans lequel Q' V' et R'₁₁ sont respectivement tels que Q V et R11 définis ci-dessus, ou
**R**_{**5**} **et R**_{**6**} représentent - (CH₂)ₘ - CW' -, m étant un entier compris entre 2 et 3 et W' ayant la même signification que W, le carbonyle ou thiocarbonyl-CW'- étant directement lié à l'azote et le radical -(CH₂)ₘ étant pour sa part lié au carbonyle ou thiocarbonyle du groupe - CW - à la condition que l'un au moins de R₁, R₆, R₇ R₈, R₂₀, représente un radical alkyl-(en C₁-C₆)-carbonyle ou un alkyl-(en C₁-C₆)-thio-carbonyle,

- le N-[2-(5-méthoxyindol-3-yl)éthyl]glutarimide (7)
- le N-[2-(2-cyclohexylcarbonyl-5-méthoxyindol-3-yl) éthyl]acétamide (8)
- le N-[2-(5-méthoxy-2-oxo-2,3-dihydroindol-3yl) éthyl]glutarimide (11) et,
- le N-[2-(6-acétyl-5-méthoxyindol-3-yl) éthyl]acétamide (12),
leurs racémiques , leurs énantiomères purs, ou leurs mélanges en toutes proportions, et leurs sels thérapeutiquement acceptables.

Par alkyle, alkoxy ou perhalogénoalkyle, on entend de manière générale des radicaux dont le reste alkyle comprend entre 1 et 6 atomes de carbone.

Il s'agit de préférence de restes alkyles linéaires ou ramifiés en C₁-C₄ plus particulièrement choisis parmi les groupes méthyle, éthyle, n-propyle, i-propyle, n-butyle ou t-butyle.

Par cycloalkyle, on entend les cycles en C₃-C₆.

Par aryle, on désigne un groupe choisi parmi les groupes phényle, thiényle, furanyle, pyridyle ou naphtyle.

Les radicaux aryles peuvent également être substitués par un ou plusieurs substituants choisis parmi les radicaux alkyle, alkoxy ou halogéno définis ci-dessus.

Par aralkyle, on entendra la combinaison d'un alkyle et d'un aryle tels que définis ci-dessus. Il s'agira de préférence du radical benzyle.

Les radicaux halogéno sont de préférence choisis parmi les atomes de fluor, de chlore de brome ou d'iode.

De préférence, les radicaux perhalogénés sont des radicaux perfluorés.

Lorsque les dérivés selon l'invention comprennent au moins un carbone asymétrique de configuration R ou S, la présente invention concerne également les racémiques des dérivés de formule générale I, ainsi que ses énantiomères purs, ou leurs mélanges en toutes proportions.

Les sels thérapeutiquement acceptables des dérivés selon l'invention sont les sels usuels de la technique, organiques ou inorganiques, notamment les chlorhydrates, les tosylates, les mésilates, les citrates ainsi que les solvates comme les hydrates ou hémihydrates des composés de formule générale I.

La présente invention concerne plus particulièrement les dérivés de formule générale I pour lesquels W représente un atome d'oxygène.

D'une manière plus avantageuse, l'un au moins des substituants R2 ou R3 est différent d'un atome d'hydrogène et représente de préférence un radical hydroxyle ou alkoxy dont le reste alkyle est en C₁-C₆, en particulier un radical méthoxy.

D'une manière préférentielle, R1, R4 et R6 représentent un atome d'hydrogène.

Parmi les dérivés préférés selon l'inventeur, R5 est avantageusement un radical alkyle en C₁-C₆, de préférence un méthyle, un éthyle, un n-propyle ou un radical perfluorométhyle, perfluoroéthyle ou perfluoropropyle, de préférence perfluoroéthyle.

D'une manière avantageuse, R7 représente un radical alkyl-(C₁-C₆)-carbonyle.

De même, Y ----- est de préférence un radical divalent de formule CR8 = et R8 représente un atome d'hydrogène ou un radical alkyl- (C₁-C₆)-carbonyle.

Lorsque R5 et R6 forment ensemble le radical (CH₂)ₘ-CW'-, le carbonyle ou thiocarbonyle - CW'- est directement lié à l'azote et le radical - (CH₂)ₘ est pour sa part lié au carbonyle ou thiocarbonyle du groupe - CW -R5.

La présente invention concerne également le procédé de préparation des dérivés de formule générale I tels que définis ci-dessus.

Les dérivés selon la présente invention peuvent être obtenus en faisant réagir l'amine correspondante de formule générale II. 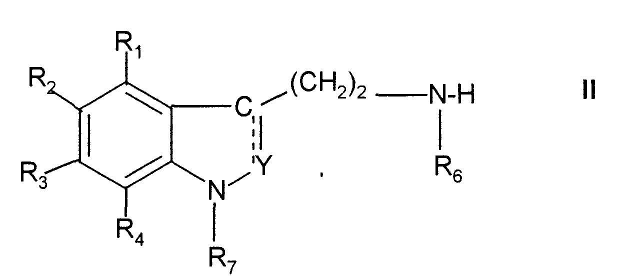 Y, R1 à R4, R6 et R7 étant définis ci-dessus, avec un agent d'acylation approprié, selon les techniques usuelles de préparation des amides, de manière à introduire le radical - CW - R5
W représentant un atome d'oxygène et R5 étant défini ci-dessus, avec l'halogénure ou l'anhydride correspondant, ou encore avec une activation de l'acide correspondant avec éventuellement un agent de couplage, telle qu'employée en synthèse peptidique.

Les dérivés pour lesquels R5 et R6 forment ensemble partie d'un cycle de formule -(CH₂)ₘ-CW'-, avec W' représentant un atome d'oxygène, sont préparés en acylant le dérivé de formule générale II pour lequel R6 représente un atome d'hydrogène avec un agent d'acylation approprié de manière à introduire le radical -CW'-(CH₂)ₘ-CW - O - alkyle, W représentant un atome d'oxygène et m étant défini ci-dessus, puis à cycliser l'amide obtenu par une réaction appropriée, par exemple par catalyse acide en présence de traces d'acide paratoluènesulfonique dans du xylène.

La transformation d'un composé de formule I dans lequel W représente un atome d'oxygène, en un composé de formule I dans lequel W représente un atome de soufre, est réalisée par le traitement par un réactif de sulfuration classique comme le pentasulfure de phosphore ou le réactif de LAWESSON.

On peut également préparer les dérivés pour lesquels Y ----- représente un radical divalent de formule 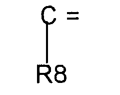 et R8 représente un radical alkyl-( C₁-C₆)-carbonyle, par hydrolyse de son précurseur cyclique de formule générale III 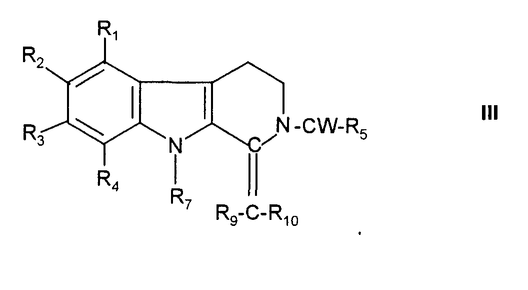 dans laquelle R1 à R5 et R7 sont définis ci-dessus, R9 et R10 représentent un atome d'hydrogène ou un reste alkyle en C₁-C₆ ou R9 et R10 forment ensemble un cycloalkyle pour obtenir le dérivé correspondant de formule I dans lequel R6 représente un atome d'hydrogène, et R8 représente un radical

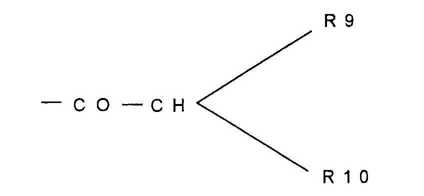

Le dérivé obtenu précédemment pourra être ensuite transformé pour obtenir un nouveau dérivé de formule générale I pour lequel R6 est différent d'un atome d'hydrogène.

D'autres caractéristiques des dérivés selon l'invention et leur procédé de préparation apparaîtront à la lumière des exemples ci-après.

### Produits de départ :

Les produits de départ, répondant aux formules générales II et III, sont disponibles dans le commerce ou peuvent être obtenus notamment selon les méthodes décrites ci-dessous :
- 5-méthoxytryptamine :
   - SUPNIEWSKI et al.CA.55, 15458 (1961).
- Mélatonine :
   - SZMUSKOVICS et al. J. Org. Chem. ,25 857 (1960),
   - Chem. and Eng. News, 45, 40 (1967).
- Analogues naphtaléniques de sérotonine et de la mélatonine :
   - ANDRIEUX J., ANKER D., MENTZER C., Chim. Thér., 57 (1966),
   - YOUS S., ANDRIEUX J., HOWELL H.E., MORGAN P.J., RENARD P., PFEIFFER B., LESIEUR D., GUARDIOLA-LEMAITRE B., J. Med.Chem., 35, 1484 (1992).
   - N-[2-(5-méthoxy-2-oxo-2,3-dihydroindol-3-yl]éthyl]acétamide :
   - SZABO-PUSZTAI K., SZABO L., Synthesis, 276 (1979).

### Exemple 1 :

### N-[2-(1-acétyl-5-méthoxyindol-3-yl)éthyl]acétamide (1)

Dans un ballon de 50 ml, on dissout la mélatonine (126 mg) dans le tétrahydrofurane (10 ml), on ajoute ensuite l'hydrure de sodium (200 mg), le chlorure d'acétyle, l'agitation est maintenue une nuit (température ambiante). Après filtration et dilution (AcOEt), la phase organique est lavée à l'eau, puis séparée sur plaque de silice. On obtient majoritairement le N-[2-(1-acétyl-5-méthoxyindol-3-yl) éthyl] acétamide (1), et un produit secondaire, le N-[2-(1-acétyl-5-méthoxyindol-3-yl) éthyl] diacétamide (10) (cf. exemple 10). N- [2-(1-acétyl-5-méthoxyindol-3-yl) éthyl] acétamide (1)
RMN ¹H : CDCl₃ : 1,96 (s, 3H, CH₃CO-Nβ) ; 2,49 (s, 3H, CH₃CO-Nα) ; 2,87 (t, 2H, CH₂); 3,56 (t, 2H, CH₂-N) ; 3,84 (s, 3H, CH₃O) ; 6,05 (s large, 1H, NH) ; 6,91 (d, 1H, H-6) ; 6,96 et 7,19 (2s, 2H, H-4 et H-2) ; 8,24 (d, 1H, H-7) ; MS (m/z) : 274(M⁺·), 215, 173, 160 (100).
Masse exacte : Calculée 274,1317
Trouvée 274,1320

### EXEMPLE 2 :

### N-[2-(2-acétyl-5-méthoxyindol-3-yl) éthyl] acétamide (2)

### Mode opératoire a

Dans un ballon de 25 ml, on dissout la 1-méthylène-2-acétyl-6-méthoxy-1,2,3,4-tétrahydro-β-carboline (100 mg) dans une solution acide (HCl, O,1 M, 10 ML) l'ensemble est chauffé à 60°C pendant une heure. Le précipité est filtre puis lavé à l'éther. On obtient ainsi le N- [2-(2-acétyl-5-méthoxyindol-3-yl) éthyl] acétamide (2).

### Mode opératoire b

Au N- [2-(5-méthoxyindol-3-yl) éthyl] diacétamide (4) (35 mg) dissous dans le dichlorométhane (3 mL) on ajoute à 0°C le réactif de Meerwein (0,15 mmole - 0,15 mL). L'ensemble est maintenu à température ambiante pendant 12 h. La solution est filtrée. On obtient un précipité rouge. Le précipité est dissous dans le méthanol (1 mL). Après 15 min de réaction on évapore le méthanol et on extrait à l'acétate d'éthyle. On obtient ainsi le N-[2-(2-acétyl-5-méthoxyindol-3-yl) éthyl] acétamide (2) R = 75 %.
RMN ¹H : CDCl₃ : 1,90 (s, 3H, CH₃CO-Nβ) ; 3,29 (t, 2H, CH₂) ; 2,72 (s, 3H, 2-CH₃CO) ; 3,40 (t, 2H, CH₂-N) ; 3,97 (s, 3H, CH₃O) : 7,11 (dd, 1H, H-6) ; 7,32 (s large, 1H, NHCO) ; 7,39 (d, 1H, H-4) ; 7,47 (d, 1H, H-7) ; 10.63 (s large, 1H, NH indole) ;
SM : m/z 274 (M⁺), 215 (100), 202, 188, 160.
Masse exacte : Calculée 274,1317
Trouvée 274,1318

### EXEMPLE 3 :

### N- [2-(2-acétyl-5-méthoxyindol-3-yl) éthyl] propionamide (3)

On reprend le mode opératoire de l'exemple 2 avec comme produit de départ la 1-methylène-2-propionyl-6-methoxy)-1,2,3,4-tétrahydro-β-carboline.
RMN ¹H :CD₃COOD₃ : 1,76 (t, 3H, CH₃ Ethyle) ; 2,23 (t, 2H, CH₂ éthyle) ; 2,72 (s, 3H, CH₃CO en position 2 indole) ; 3,40 (t, 2H, CH₂); 3.57 (q, 2H, CH₂ N) ; 3,95 (s, 3H, CH₃O); 7,10 (dd, 1H, H-6) ; 7,20 (s large, 1H, NHCO); 7,40 (d, 1H, H-4) ; 7,5 (d, 1H, H-7) ; 10,6 (s large, 1H, NH indole) ;
SM : m/z 288 (M⁺ ·), 245, 215 (100), 202, 188.
Masse exacte : Calculée 288,1473
Trouvée 288,1470

### EXEMPLE 4 :

### N- [2-(5-méthoxyindol-3-yl) éthyl] diacétamide (4)

### Mode opératoire a

A la mélatonine (500 mg) dissoute dans le benzène (50 ml), on ajoute sous agitation l'anhydride acétique (7 ml). L'ensemble est chauffé 72 h au reflux du benzène. Le solvant est évaporé, le brut est repris à l'eau pui neutralisé par une solution de carbonate de sodium (pH > 8). Après extraction (dichlorométhane), lavage (eau) et séchage (sulfate de magnésium), le brut est flash-chromatographié (éluant AcOEt). On obtient le N- [2-(5-méthoxyindol-3-yl) éthyl] diacétamide (4) (300 mg, Rdt 50 %).

### Mode opératoire b

A la mélatonine (380 mg) on ajoute sous agitation l'anhydride acétique (3 mL). L'ensemble est chauffé 4 h à 145°C. Après évaporation de l'anhydride acétique. Le brut est flash chromatographié (éluant AcOEt / Ether de pétrole 50/50). On élue successivement :
- le N- (2-(5-méthoxyindol-3-yl) éthyl] diacétamide (4) (180 mg, Rdt 40 %), RMN ¹H : DMSO D₆ : 2,30 (s, 6H, 2(CH₃CO)) ; 2,93 (t, 2H, CH₂) ; 3,81 (s, 3H, CH₃O) ; 3,89 (t, 2H, CH₂-N) ; 6,76 (d, 1H, H-6) ; 7,02 et 7,07 (2s, 2H, H-2 et H-4) ; 7,22 (d, 1H, H-7) ; 10,48 (s large, 1H, NH) ;
SM : m/z 274 (M⁺), 173 (100), 160, 145, 77.
Masse exacte : Calculée 274,1317
Trouvée 274,1320

### EXEMPLE 5 :

### N- [2-(2-acétyl-indol-3-yl) éthyl] acétamide (5)

On reprend le mode opératoire de l'exemple 2 avec comme produit de départ la 1-méthylène-2-acétyl-1,2,3,4-tétrahydro-β-carboline.
RMN ¹H : CDCl₃ : 1,88 (t, 3H, CH₃CO) ; 2,58 (s, 3H, CH₃CO en position 2 indole) ; 3,27 (t, 2H, CH₂ en position 3 indole) ; 3,53 (t, 2H, CH₂-N) ; 6,68 (s large, 1H, NHCO) ; 7,08 (t, 1H, H-7) ; 7,32 (m, 2H, H-5 et 6) ; 7,61 (d, 1H, H-4) ; 10 (s large, 1H, NH indole) ;
SM : m/z 244 (M⁺·), 185 (100), 172, 158, 130.

### EXEMPLE 6 :

### N-[2-(1-acétyl-2-oxo-5-méthoxy-2,3-dihydroindol-3-yl)éthyl] acétamide (6)

A la N-[2-(5-méthoxy-2-oxo-2,3-dihydroindol-3-yl)éthyl] acétamide (120 mg) dissoute dans le benzène (5ml), on ajoute sous agitation l'anhydride acétique (0,5 ml). L'ensemble est chauffé 1 h au reflux du benzène. Le solvant est évaporé, le brut est séparé sur plaque de silice. On obtient ainsi le N- [2-(1-acétyl-2-oxo-5-méthoxyindol-3-yl) éthyl] acétamide (6).
RMN ¹H : CD₃COCD₃ : 1,84 (s, 3H, CH₃CO-Nβ) ; 2,30 et 2,94 (2m, 2H, CH₂), 2,57 (s, 3H, CH₃CO-Nα) ; 3,25 et 3,47 (2m, 2H, CH₂-N) ; 3,76 (t, 1H, H-3) ; 3,80 (s, 3H, CH₃O) ; 6,57 (d, 1H, H-6) ; 7,09 (s, 1H, H-4) ; 8.03 (d, 1H, H-7) ; 7,22 (s large, 1H, NH).

### EXEMPLE 7 :

### N- [2-(5-méthoxyindol-3-yl) éthyl] glutarimide (7)

On chauffe à 175°C un mélange de 5-méthoxytryptamine (420 mg) et du glutarate de diéthyle (460 mg), pendant 18 h. La séparation sur colonne (éluant AcOEt) fournit l'amide ester correspondant.

Ce dernier est traité par une trace d'acide paratoluènesulfonique dans du Xylène, l'éthanol formé est éliminé à l'aide d'un Dean Stark. Après 9 h de reflux et séparation sur plaque de silice on obtient le N-[2-(5-méthoxy-indol-3-yl)éthyl]glutarimide (7).
RMN ¹H :CDCl₃ : 1,88 (m, 2H, CH₂ en β des 2 CO) ; 2,62 (t, 4H, CH₂ en α des CO) ; 2,93 (t, 2H, CH₂ en position 3 indolique) ; 3,90 (s, 3H, OCH₃) ; 4,05 (t, 2H, CH₂-NCO) ; 6,87 (dd, 1H, H-6) ; 7,03 (s, 1H, H-4) ; 7,20 (d, 1H, H-7) ; 8,0 (s large, 1H, N-H indole).
Spectre de masse : m/z : 286 (M⁺·), 173 (100), 160.
Masse exacte : Calculée 286,1317
Trouvée 286,1310

### EXEMPLE 8 :

### N- [2-(2-cyclohexylcarbonyl-5-méthoxyindol-3-yl) éthyl] acétamide (8)

En reprenant le mode opératoire de l'exemple 2, on obtient le N- [2-(2-cyclohexylcarbonyl-5-méthoxyindol-3-yl) éthyl] acétamide (8).
RMN ¹H : CDCl₃ : entre 1,30 et 1,80 (massif complexe, 10H cyclohexyle) ; 1,91 (s, 3H, NCOCH₃) ; 3,30 (t, 2H, CH₂ en position 3 indolique) ; 3,59 (t, 2H, CH₂-NCO) ; 3,81 (s, 3H, OCH₃) ; 6,72 (s large, NHCO) ; 6,98 (d, 1H, H-6) ; 7,01 (s, 1H, H-4) ; 7,27 (d, 1H, H-7) ; 9,75 (s large, 1H, N-H indole).
Spectre de masse : m/z : 342 (M⁺·), 283 (100), 268, 188.

### EXEMPLE 9 :

### N- [2-(2-acétyl-5-méthoxyindol-3-yl) éthyl] diacétamide (9)

Le N- [2-(2-acétyl-5-méthoxyindol-3-yl) éthyl] diacétamide (9) est un produit secondaire de la réaction d'acylation de la mélatonine selon le procédé de l'exemple 4, mode opératoire b, isolé par flash chromatographie.
RMN ¹H : CDCl₃: 2,39 (s, 3H, CH₃CO-Nβ) ; 2.58 (s, 6H, CH₃CO-Nα) ; 3.35 (t, 2H, CH₂-Ar) ; 3,90 (s, 3H, CH₃O) ; 3,90 (t, 2H, CH₂-Nβ) ; 6,98 (dd, 1H, H-6) ; 7,12 (d, 1H, H-4) ; 7,29 (d, 1H, H-7); 8,85 (s large, 1H, NH).

### EXEMPLE 10 :

### N- [2-(1-acétyl-5-méthoxyindol-3-yl) éthyl] diacétamide (10)

RMN ¹H : CDCl₃: 2,39 (s, 3H, CH₃CO-Nβ) ; 2,58 (s, 6H, CH₃CO-Nα) ; 2,94 (t, 2H, CH₂); 3,89 (s, 3H, CH₃O) ; 3,93 (t, 2H, CH₂-N); 6,95 (dd, 1H, H-6) ; 7,13 (d, 1H, H-4); 7,22 (s, 1H, H-2) ; 8,3 (d, 1H, H-7); SM : m/z 316 (M^{+.}), 215, 173(100), 160.

### EXEMPLE 11 :

### N-[2-(5-méthoxy-2-oxo-2,3-dihydroindol-3-yl)éthyl]glutarimide (11)

Dans un ballon, on dissout 145 mg N-[2-(5-méthoxyindol-3-yl)éthyl]glutarimide dans du DMSO (30ul), on ajoute sous agitation du HCl concentré (72ul). L'ensemble est agité une nuit à température ambiante. Le brut est neutralisé par NH₃, puis extrait par l'AcOEt. Après séparation sur plaque de silice on obtient le N-[2-(5-méthoxy-2-oxo-2,3-dihydroindol-3-yl)éthyl]glutarimide.
RMN ¹H (CDCl₃) : 1,82 (m, 2H, CH₂ en b des 2 CO); 2,18 (2m, 2H, CH₂ ), 2,52 (t, 4H, CH₂ en α des CO); 3,47 et 3,85 (2m, 2H, CH₂-N); 3.78 (s, 3H, OCH₃); 4,09 (t, 1H, H-3); 6,97 (m, 2H, H-6, H-7); 6,91 (s, 1H, H-4).

### EXEMPLE 12 :

### N-[2-(6-acétyl-5-méthoxyindol-3-yl)éthyl]acétamide (12)

Le N-[2-(6-acétyl-5-méthoxyindol-3-yl)éthyl]acétamide est préparé par hydrolyse alcaline du N-[2-(6-acétyl-1-carbéthoxy-5-méthoxyindol-3-yl)éthyl]acétamide dans la potasse alcoolique.
RMN ¹H : CDCl₃ : 1,59 (s, 3H), 2,61 (s, 3H); 2.88 (t, 2H); 3.43 (q, 2H); 3.90 (s. 3H); 6.25 (s large, 1H); 6,95 (s. 1H); 7.06(s, 1H); 7,76 (s. 1H);
MS (m/z) : 274 (M^{+.}), 215, 202(100)

### EXEMPLE 14 :

### N-[2-(1-carbéthoxy-2-acétyl-5-méthoxyindol-3-yl)éthyl]acétamide (14)

Dans un ballon (25ml), on dissout la 1-Méthylène-2-acétyl-9-carbéthoxy-6-méthoxy-bCarboline (100mg) dans une solution acide (HCl, 0,1M, 10ml) l'ensemble est chauffé à 60°C pendant une heure. Le précipité est filtré puis lavé à l'ether. On obtient ainsi le N-[2-(1-carbéthoxy-2-acétyl-5-méthoxyindol-3-yl)éthyl]acétamide.
RMN ¹H : CDCl₃ : 1,45 (t,3H); 1,92 (s, 3H); 2,45 (s. 3H); 2,84 (t. 2H); 3,49 (t, 2H); 3,83 (s, 3H); 4,47 (q, 2H); 7,0-7,06 (m, 3H); 7,92 (d, 1H);
MS (m/z) : 346 (M^{+.}), 287, 215, 202 (100)

### EXEMPLE 15 :

### N-[2-(2-acétyl-6-éthyl-5-méthoxyindol-3-yl)éthyl]acétamide (15)

Dans un ballon (25ml), on dissout la 1-Méthylène-2-acétyl-7-éthyl-6-méthoxy-βCarboline (100mg) dans une solution acide (HCl, 0,1M, 10ml) l'ensemble est chauffé à 60°C pendant une heure. Le précipité est filtré puis lavé à l'ether. On obtient ainsi le N-[2-(2-acétyl-6éthyl-5-méthoxyindol-3-yl)éthyl]acétamide
RMN ¹H : CDCl₃ : 1,22 (t,3H); 1,91 (s, 3H); 2,60 (s, 3H); 2,72 (q, 2H); 3,27 (t, 2H, CH₂); 3,52 (t, 2H); 3,86 (s, 3H); 6,97 (t large, 1H); 7,15 (s, 1H); 7,31 (s, 1H);
MS (m/z) : 302 (M^{+.}), 243 (100), 259, 230, 216

### EXEMPLE 16 :

### N-[2-(1-acétyl-5-méthoxyindolin-3-yl)éthyl]acétamide (16)

A une solution refroidie à 0°C de mélatonine (1mmol) dans l'acide trifluoroacétique (2mL), on additionne goutte à goutte le borane ( 2 éq, solution 1M dans le THF). L'ensemble est agité 30 min à 0°C, puis addition goutte à goutte d'eau (1,5mL), le milieu est laissé agité 1h à température ambiante. L'ensemble est ensuite amené à pH=10 à l'aide d'une solution de potasse 2N, puis extrait au dichlorométhane. Le brut est séparé sur colonne de silice flash (Acétone/dichlorométhane/méthanol-25/75/5), on obtient 110mg de N-[2-(5-méthoxyindolin-3-yl)éthyl]acétamide qui est ensuite acétylée par l'anhydride acétique dans la pyridine pour conduire au N-[2-(1-acétyl-5-méthoxyindolin-3-yl)éthyl]acétamide.
RMN ¹H : CDCl₃ :1,65 (m, 1H); 1,93 (s, 3H); 1,9 (m, 1H); 2,12 (s, 3H); 3,31 (m, 3H); 3,63 (m, 1H); 3,68 (s, 3H); 4,11 (2m, 1H); 6,49 (s large, 1H); 6.66 (m, 2H); 8,02 (d, 1H)
SM : m/z 276 (M^{+.}), 272, 204, 174, 160, 148(100),

### EXEMPLE 17 :

### N-[2-(1-acétyl-6-chloro-5-méthoxyindolin-3-yl)éthyl]acétamide (17)

A une solution de N-[2-(1-acétyl-5-méthoxyindolin-3-yl)éthyl]acétamide (0.29mmol) dans l'acétonitrile (1,5mL) on ajoute le bis trifluoroacétoxyiodobenzéne (PIFA 1.2 éq). le milieu est laissé sous agitation 1 min à température ambiante. On additione ensuite une solution saturée de chlorure de sodium (0,5mL), aprés 15min le tout est dilué avec du dichlorométhane (10mL), séché sur sulfate de magnésium. Le brut obtenu après évaporation est chromatographié sur colonne de silice flash (Méthanol/dichlorométhane-5/95). on obtient ainsi le N-[2-(1-acétyl-6-chlore-5-méthoxyindolin-3-yl)éthyl]acétamide (70mg)
RMN ¹H : CDCl₃ :1,65 (m, 1H); 1,91 (s, 3H); 1,9 (m, 1H); 2,12 (s, 3H); 3,31 (m, 3H); 3,63 (m, 1H); 3,80 (s, 3H); 4,11 (2m, 1H); 6,28 (s large, 1H); 6,75 (s, 1H); 8,15 (d, 1H)

### EXEMPLE 18 :

### N-[2-(1-benzyloxycabonyl-5-méthoxyindolin-3-yl)éthyl] diacétamide (18)

La préparation du N-[2-(5-méthoxyindolin-3-yl)éthyl]acétamide est réalisée par la méthode décrite pour la synthèse de la N-[2-(1-acétyl-5-méthoxyindolin-3-yl)éthyl]acétamide. Le N-[2-(1-benzyloxycabonyl-5-méthoxyindolin-3-yl) éthyl] acétamide intermédiaire est préparé en faisant réagir la N-[2-(5-méthoxyindolin-3-yl) éthyl]acétamide sur le chloroformiate d'éthyle dans le dichlorométhane à 0°C en présence de triéthylamine. L'acylation finale s'effectue avec de l'anhydride acétique, au reflux du toluène pendant 24h, et permet d'obtenir le N-[2-(1-triluorométhanesulfonyl-5-méthoxyindolin-3-yl) éthyl]diacétamide.
RMN ¹H : CDCl₃ : 1,96 et 2,11 (2m, 2H); 2;32 (s, 6H); 3,28 (m, 1H); 3,53 (m, 1H); 3,71 (s, 3H); 3,74-3,80 (m, 2H); 4,13 (m, 1H); 5,20 (m, 2H); 6,72 (m, 2H); 7,35 et 7,8(m, 6H)
¹³C(DEPT) : 26,1(CH₃); 33,7(CH₂); 37,4(CH); 42,1(CH₂); 37,8(CH₂); 55,3(CH₃); 66,6(CH₂); 110,3(CH); 112,4(CH); 115,1(CH); 127,9(CH); 128,0(CH); 128,6(CH); 134,4(C); 135,7(C); 136,2(C); 155,6(C); 170,0(CO); 172,6(CO).

### EXEMPLE 19 :

### N-[2-(1-triluorométhanesulfonyl-5-méthoxyindolin-3-yl)éthyl]diacétamide (19)

La préparation du N-[2-(5-méthoxyindolin-3-yl)éthyl]acétamide est réalisée par la méthode décrite pour la synthèse de la N-[2-(1-acétyl-5-méthoxyindolin-3-yl)éthyl]acétamide. Le N-[2-(1-triluorométhanesulfonyl-5-méthoxyindolin-3-yl) éthyl] acétamide intermédiaire est préparé en faisant réagir la N-[2-(5-méthoxyindolin-3-yl)éthyl]acétamide sur l'anhydride trifluorométhanesulfonique dans le dichlorométhane à -78°C en présence de triéthylamine. L'acylation finale s'effectue avec de l'anhydride acétique, au reflux du toluène pendant 24h, et permet d'obtenir le N-[2-(1-triluorométhanesulfonyl-5-méthoxyindolin-3-yl) éthyl]diacétamide.
RMN ¹H : CDCl₃ : 2.01 (m, 2H); 2,41 (s, 6H); 3,42 (m, 1H); 3,66 (m, 1H); 3,79 (s. 3H); 3,86 (m, 1H); 3,95 (m, 1H); 4,36 (t, 1H); 6,79 (m, 2H); 7,33 (d, 1H)

### EXEMPLE 20 :

### N-[2-(6-(1-acétyl-3-(N,N-diacétyl-2-aminoéthyl)-5-méthoxyindolin-2-yl)-5-méthoxyindol-3-yl)éthyl]diacétamide (20)

Le produit est préparé selon la méthode décrite par Laronze et coll. (Bull. Soc. Chim. Fr. 1966, vol 133, p39-50), puis acétylation selon le mode opératoire décrit pour la N-[2-(5-méthoxyindol-3-yl)éthyl]diacétamide
RMN ¹H : CDCl₃ : 2,03 (s, 3H); 2,37 (s, 12H); 2,45 (m, 2H); 2,96 (t, 2H); 3,0 (m, 1H); 3,73 (s, 3H); 3,78 (m, 4H); 4,00 (s,3H); 5,59 (s, 1H); 6,64 (s,1H); 6,70 (dd,1H):6,88 (s, 1H); 6,93 (d, 1H); 7,19 (s, 1H); 8,25 (d, 1H); 8,37 (s large,1H)
MS (m/z) : 590 (M^{+.}), 345, 215, 173(100)

### EXEMPLE 22 :

### N-[2-(1-carbéthoxy-2-acétyl-5-méthoxyindolin-3-yl)éthyl]acétamide (22)

Dans un ballon (25 ml), on dissout le produit N-[2-(1-carbéthoxy-2-acétyl-5-méthoxyindol-3-yl)éthyl]acétamide (100 mg) dans de l'ethanol (10 ml). on ajoute 100 mg de Pd(OH)₂, l'ensemble est agité 12 h sous pression normale d'hydrogène. On obtient après filtration le N-[2-(1-carbéthoxy-2-acétyl-5-méthoxyindolin-3-yl)éthyl]acétamide
RMN ¹H : CDCl₃ : 1,27 (s large, 3H) ; 1,50 (m, 1H) ; 1,92 (m. 7H) ; 3,45 (m. 2H) ; 3,78 (s, 3H) ; 4,30 (s large, 2H) ; 4,83 (d, 1H) ; 6 (s large, NH) ; 6,77 (m, 2H) ; 7.79 (s large, 1H).

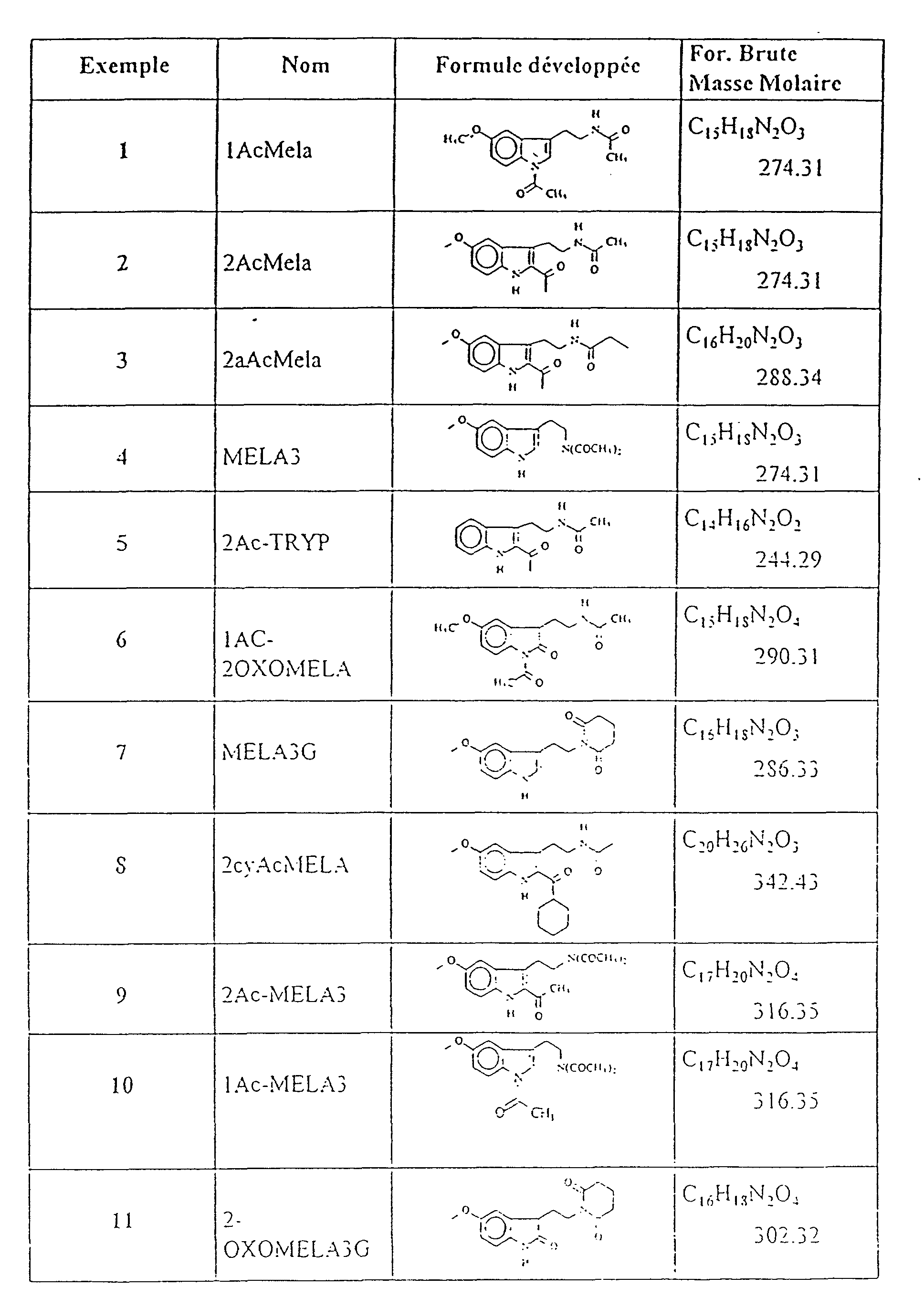

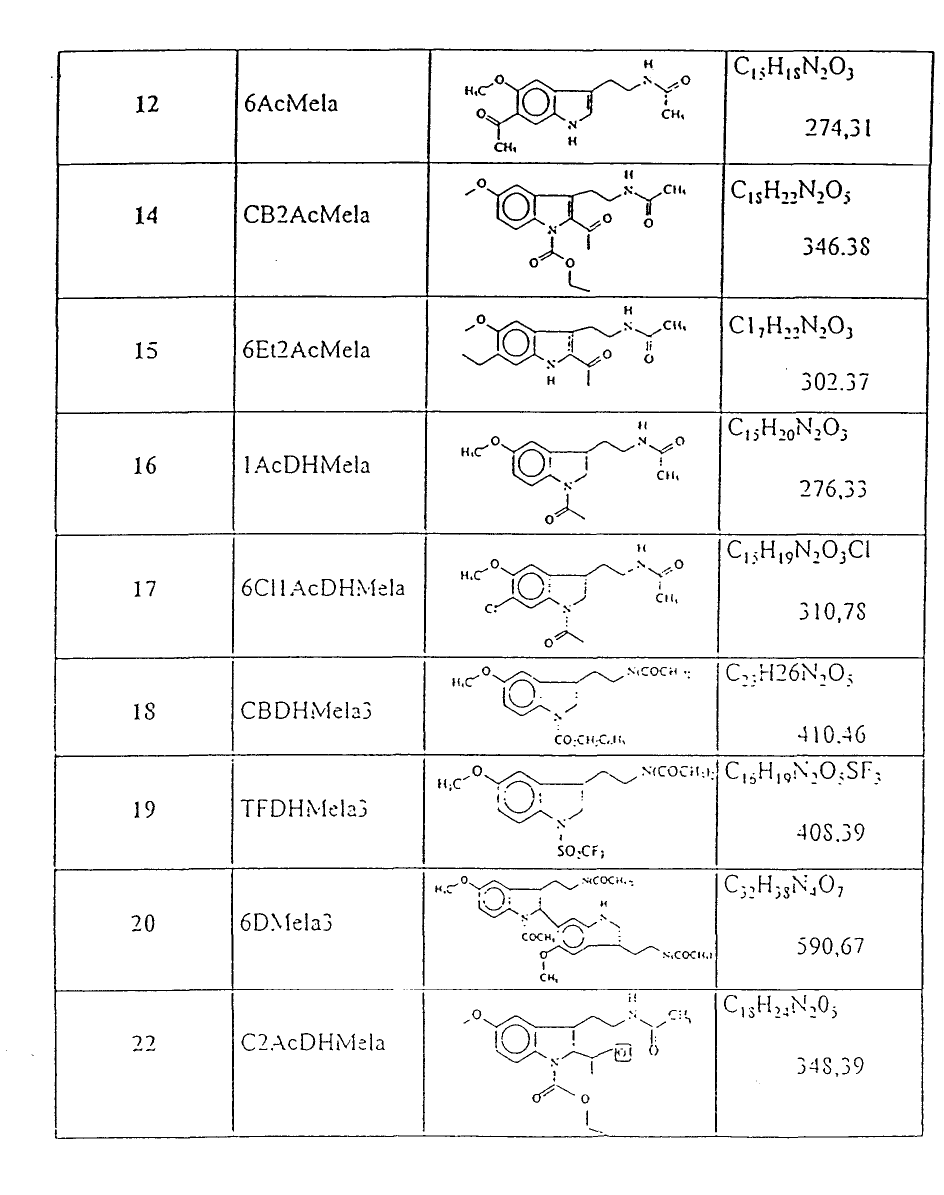

### ACTIVITE BIOLOGIQUE

Les effets hypnotiques et sédatifs des dérivés selon l'invention, préparés ci-dessus (dont les résultats des tests sont indiqués dans le Tableau 1 ci-après), ont été comparés à ceux de deux produits de référence, le diazépam et la mélatonine, chez des poussins de souche chair label JA657, âgés de 10 à 15 jours. Les animaux sont soumis à des programmes d'éclairement alterné comportant 12 h d'obscurité (20 h à 8 h) et 12 h d'éclairement (8 h à 20 h). La température d'ambiance est de 25°C pendant la première semaine d'élevage des poussins et de 22°C à partir de la deuxième semaine. Pendant la journée, l'éclairement est assuré par une lampe de 100 W, placée à 30 cm au-dessus du plancher du vivarium. Pendant les tests, les poids vifs des poussins ont varié entre 95 et 155 g. Les tests sont réalisés à partir de 14 h. Les poussins sont allotés par groupes de 3 dans des vivariums identiques de 30 cm x 50 cm x 30 cm. Les produits testés sont administrés par voie intramusculaire (1M) dans le muscle pectoral majeur, en solution hydro-éthanolique (mélange éthanol / eau distillée, 50/50, V/V), à raison de 0,2 ml de solution éthanolique pour 100 g de poids vif. Les doses administrées pour les produits testés (nouveaux composés de l'invention et substances de référence) sont équimolaires (2 µM/100 g de poids vif). Le placebo correspond à 0,2 ml du mélange éthanol/eau distillée (aa). L'éthanol étant utilisé comme solvant, son effet a été comparé préalablement à celui du soluté physiologique (soluté NaCl à 0,9 p. 100) ou de l'eau distillée.

Les solutions hydro-éthanoliques des produits testés ont été préparées extemporanément par dilution successive d'une solution mère, obtenue à partir de 20 µM de produit, exactement pesées, additionnées de 1 ml d'éthanol pur, agitées aux ultrasons, puis complétées à 2 ml avec 1 ml d'eau distillée pour préparation injectable. Dans le Tableau 1 sont présentés les résultats obtenus après administrations 1M de 2 µMoles des produits testés, en solution dans 0,2 ml du mélange éthanol/eau distillée, pour 100 g de poids vif. Pour chaque poussin, le volume injecté est ajusté, en fonction du poids vif réel, à 0,2 ml pour 100 g de poids vif.

Les paramètres observés sont l'activité locomotrice et l'état de veille des poussins pendant 2 h, soit l'équivalent de 6 cycles théoriques veille-sommeil du poussin de cet âge. Ils sont enregistrés par caméra vidéo pendant 120 minutes.

Cinq stades de vigilance ont été définis :
- stade 1 : veille active ;
- stade 2 : animal couché, maintien de la tête avec tonicité, oeil ouvert ;
- stade 3 : sommeil léger, animal assoupi : oeil fermé avec ouverture intermittente, posture immobile non modifiée par la stimulation ;
- stade 4 : sommeil profond couché : relâchement du cou, posture caractéristique tête sous l'aile ou en arrière ;
- stade 5 : sommeil debout : oeil fermé, immobile, tête tombante (catatonique).

Ces cinq stades correspondent approximativement aux stades de vigilance et de sommeil définis à l'examen des tracés électro-encéphalographiques dans cette espèce. La correspondance est la suivante :
. sommeil profond couché : stade 4 = "slow wave sleep" (SWS)
. sommeil debout : "sleep-like state 1" (SLSI)

Le stade 3, assoupi, pourrait correspondre à des phases de sommeil paradoxal, avec agitation de la tête, par exemple.

L'observation des poussins est réalisée par un observateur entraîné avec un contrôle vidéo continu pendant au moins 1 heure après le réveil des animaux.

Deux stimuli ont été utilisés pour confirmer les observations du comportement des poussins à intervalles réguliers :
- le bruit causé par le choc d'un objet en plastique sur la vitre du vivarium. comparable à celui du bec d'un poussin sur la vitre, correspond à un stimulus modéré. Il est pratiqué à chaque période d'observation (soit toutes les 5 minutes) ;
- et la présentation d'une mangeoire métallique remplie avec l'aliment habituel, laissée 2 minutes dans le vivarium. Il s'agit d'un stimulus puissant faisant appel à la vision, l'ouïe et l'odorat. Elle est pratiquée toutes les 15 minutes, c'est-à-dire 6 fois, au moins, à chaque essai.

Le réveil est défini par l'apparition du comportement élaboré conscient de recherche et consommation de nourriture ou de boisson.

Le Temps de Sommeil (TS) est défini par la somme des durées des phases de sommeil léger (stade 3), sommeil profond (stade 4) et sommeil debout (stade 5). Le Temps de Sédation correspond à la somme des temps différents de la veille active pendant la période d'observation de 120 minutes.

Le Temps d'Assoupissement (TA) est égal (à 1 minute près) au temps nécessaire au passage d'état de veille active (stade 1) à un état non vigile (stades 3, 4 et 5).

Les effets hypnotiques et sédatifs des produits d'essai sur l'activité diurne de poussins, âgés de 10 à 15 jours, soumis à un programme d'éclairement permanent de la naissance au 6ème jour, puis à un programme d'éclairement alterné de 12 h de jour (8 h - 20 h) et 12 h d'obscurité (20 h - 8 h) sont reportés au Tableau 1 ci-après.

Chaque série d'essais débute à 14 h. Pour chaque produit testé, plusieurs séries de mesures ont été réalisées sur des lots de 3 animaux, chaque valeur indiquée étant la moyenne de 1 ou plusieurs lots de 3 animaux.

Ont été mesurées les valeurs suivantes :
- TA : temps d'assoupissement égal au temps nécessaire pour passer de l'état de veille active à un état non vigile ;
- TS : temps de sommeil, égal à la durée de la période de sommeil allant de l'endormissement au réveil ;
- T. Sédation : somme des temps différents de la veille active pendant la période d'observation de 120 minutes.

**TABLEAU IV**

| COMPOSE | DOSE (µM/100 G) | TA (minutes) | TS (minutes) | T. Sédation (minutes) |
|---|---|---|---|---|
| 1 | 2 | 5 - 8 | 55 - 85 | 80 - 95 |
| 2 | 2 | 4 - 5 | 49 - 92 | 89 - 97 |
| 4 | 2 | 6 - 7 | 55 - 97 | 95 - 102 |
| 6 | 2 | 5 - 10 | 70 - 75 | 75 - 90 |
| 9 | 2 | NA - 15 | 0 - 30 | 37 - 60 |
| 16 | 2 | 13 - 15 | 20 - 45 | 38 - 73 |
| 20 | 2 | 4 - 5 | 25 - 68 | 40 - 68 |
| Mélatonine | 2 | NA | 0 | 65 - 105 |
| Diazépam | 2 | 2 - 5 | 81 - 100 | 95 - 115 |
| Placébo | 0 | NA | 0 | 30 - 65 |
| NA = non applicable, les animaux restent vigiles. | | | | |

Les résultats obtenus montrent, pour les dérivés selon l'invention. un effet hypnotique supérieur à celui des produits de référence (mélatonine) et équivalent à celui du diazépam.

Les dérivés selon l'invention sont donc particulièrement avantageux pour le traitement de maladies liées aux désordres de l'activité de la mélatonine.

La présente invention concerne donc les dérivés de formule générale I, tels que définis précédemment pour leur utilisation en thérapie, notamment pour le traitement de la dépression et des désordres psychiatriques, en particulier le stress, l'andété, la dépression, l'insomnie, la schizophrénie, les psychoses, l'épilepsie, mais également pour le traitement des troubles du sommeil liés aux voyages ("jet lag"), des maladies neurodégénératives du système nerveux central comme la maladie de Parkinson ou la maladie d'Alzheimer, pour le traitement de cancers, ou encore comme contraceptif, ou comme analgésique.

Les analogues mélatoninergiques selon l'invention sont également utiles pour le traitement de l'hyperplasie bénigne de la prostate, des cancers de la peau, les affections de la peau comme le psoriasis, l'acné, les mycoses, du glaucome, ainsi que pour augmenter les résistances immunitaires.

Ils sont également utiles pour prévenir les symptômes de la ménopause, les syndrômes pré-menstruels, les effets du vieillissement et la mort subite du nouveau-né.

Ils sont également utiles en application vétérinaire pour réguler les naissances chez les animaux ruminants.

La présente invention concerne donc également les compositions pharmaceutiques adaptées pour l'administration des dérivés de formule générale 1, notamment par voie orale, parentérale, rectale, sous la forme de capsules, comprimés, gélules, solutions buvables, solutions injectables, y compris les formes retard et les pansements à libération prolongée pour l'administration transdermique du principe actif, les spray nasals, ou les formulations topiques (crème, émulsion, etc.), comprenant un dérivé de formule générale 1 selon l'invention et au moins un excipient acceptable pharmaceutiquement.

Les compositions pharmaceutiques selon l'invention sont avantageusement dosées pour délivrer le principe actif en une seule "prise".

Pour une administration par voie orale, les doses unitaires efficaces sont comprises entre 0,1 µg et 500 mg.

Pour une administration intra-veineuse, les doses unitaires efficaces sont comprises entre 0,1 µg et 100 mg.

Les analogues mélatoninergiques selon l'invention sont également utiles en cosmétique, notamment pour la protection de la peau contre le vieillissement, mais également contre la chute des cheveux.

La présente invention concerne donc également une composition cosmétique comprenant un dérivé de formule générale I selon l'invention.

Les compositions cosmétiques selon l'invention sont formulées d'une manière appropriée, pour leur application topique, notamment sous la forme de pommades, crèmes, émulsions, onguents, lotions, etc.

## Revendications

1. Dérivés choisis dans le groupe constitué par :
- les dérivés de la formule générale I 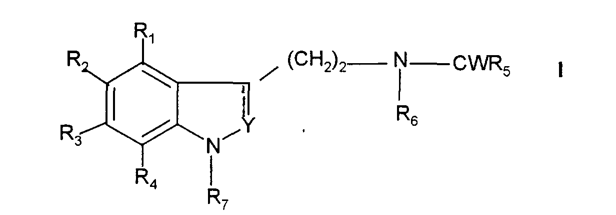
dans laquelle
**W** représente un atome d'oxygène ou de soufre ou un radical =NR₁₂, R₁₂ étant un atome d'hydrogène, un radical alkyle en C₁-C₆, aryle, aralkyle dont le reste alkyle est en C₁-C₆, cycloalkyle en C₃-C₆,
**Y ---** représente un radical de formule CR₈ =, CW₁-, ou CHR₂₀, W₁ ayant le même définition que W ci-dessus,
**R**_{**1**} **à R**_{**4**} représentent indépendamment l'un de l'autre un atome d'hydrogène, un radical hydroxyle, un radical alkyle en C₁-C₆, alkoxy dont le reste alkyle est en C₁-C₆, aryloxy, aralkoxy dont le reste alkyle est en C₁-C₆, halogéno ou nitro,
**R**_{**5**} représente un atome d'hydrogène, un radical alkyle en C₁-C₆, aryle, aralkyle dont le reste alkyle est en C₁-C₆, un radical perhalogéno-alkyle en C₁-C₆, un radical amino, alkyl-( en C₁-C₆)-amino ou dialkyl-(en C₁-C₆)-amino, ou un radical alkoxy dont le reste alkyle est en C₁-C₆.
**R**_{**6**} **et R**_{**20**} représentent indépendamment l'un de l'autre un atome d'hydrogène, un radical hydroxyle, un radical alkyle en C₁-C₆, cycloalkyle en C₃-C₆, alkoxy dont le reste alkyle est en C₁-C₆, aryloxy, aralkoxy dont le reste alkyle est en C₁-C₆, alkyl-(en C₁-C₆) thio, arylthio, aralkyl-(en C₁-C₆)-thio, halogéno, nitro ou une chaîne aliphatique insaturée, alkenyle en C₂-C₆, alkynyle en C₂-C₆, alkyle en C₁-C₆, aryle, aralkyle dont le reste alkyle est en C₁-C₆, un radical perhalogéno-alkyle en C₁-C₆, un radical amino, alkyl-(en C₁-C₆)-amino, dialkyl-(en C₁-C₆)-amino, arylamino, diarylamino, aralkylamino dont le reste alkyle est en C₁-C₆, un radical de la forme CV - R₁₁ ou QCVR₁₁, dans lequel V représente un atome d'oxygène ou de soufre ou un radical imine = N - R₁₂, R₁₁ a l'une des significations de R₁.
dans lesquels le terme aryle représente un groupe phényle, thiènyle, furanyle, pyridyle ou naphtyle, éventuellement substitué par un ou plusieurs substituants choisis parmi les radicaux alkyle en C₁-C₆, alkoxy dont le reste alkyle est en C₁-C₆ ou halogèno,
**Q** représente un atome d'oxygène ou de soufre,
**R**_{**7**} a l'une des significations de R₁ sauf qu'il ne peut être hydroxyle, ou il peut représenter un radical SO₂R₂₆, R₂₆ étant un radical alkyle en C₁-C₆ ou haloalkyle en C₁-C₆, notamment CF₃,
**R**_{**8**} a l'une des significations de R₁ et peut également représenter un atome d'halogène (chlore, brome, iode, fluor), un groupe Q'-CV'-R'₁₁ dans lequel Q' V' et R'₁₁ sont respectivement tels que Q V et R₁₁ définis ci-dessus, ou
**R**_{**5**} **et R**_{**6**} représentent - (CH₂)ₘ - CW' -, m étant un entier compris entre 2 et 3 et W' ayant la même signification que W, le carbonyle ou thiocarbonyle -CW'-étant directement lié à l'azote et le radical -(CH₂)ₘ étant pour sa part lié au carbonyle ou thiocarbonyle du groupe -CW-,
à la condition que l'un au moins de R₁, R₆, R₇, R₈, R₂₀, représente un radical alkyl-(en C₁-C₆)-carbonyle ou un alkyl-(en C₁-C₆)-thio-carbonyle,
- le N-[2-(5-méthoxyindol-3-yl)éthyl]glutarimide (7),
- le N-[2-(2-cyclohexylcarbonyl-5-méthoxyindol-3-yl)éthyl]acétamide (8),
- le N-[2-(5-méthoxy-2-oxo-2, 3-dihydroindol-3-yl)éthyl]glutarimide (11), et
- le N-[2-(6-acétyl-5-méthoxyindol-3-yl)éthyl]acétamide (12),
leurs racémiques , leurs énantiomères purs, ou leurs mélanges en toutes proportions, et leurs sels thérapeutiquement acceptables.

2. Dérivés selon la revendication 1, **caractérisés en ce que** l'un au moins des substituants R₂ ou R₃ est différent d'un atome d'hydrogène et représente de préférence un radical hydroxyle ou alkoxy dont le reste alkyle est en C1-C6, en particulier un radical méthoxy.

3. Dérivés selon l'une des revendications 1 ou 2, **caractérisés en ce que** R1 et R4 représentent un atome d'hydrogène.

4. Dérivés selon les revendications 1 à 3, **caractérisés en ce que** R5 est un radical alkyle en C₁-C₆, de préférence un méthyle ou un éthyle, ou un radical perfluorométhyle, perfluoroéthyle ou perfluoropropyle, de préférence perfluoroéthyle.

5. Dérivés selon l'une des revendications 1 à 4, **caractérisés en ce que** R7 représente un radical alkyl-(C₁-C₆)-carbonyle.

6. Dérivé selon l'une des revendications 1 à 5, **caractérisés en ce que** Y ----- représente un radical divalent de formule CR8 = et R8 représente un atome d'hydrogène ou un radical alkyl-(C₁-C₆)-carbonyle.

7. Dérivés selon la revendication 1, sélectionnés parmi les composés suivants :
N-[2-(1-acétyl-5-méthoxyindol-3-yl) éthyl] acétamide (1),
N- [2-(2-acétyl-5-méthoxyindol-3-yl) éthyl] acétamide (2)
N- [2-(2-acétyl-5-méthoxyindol-3-yl) éthyl] propionamide (3)
N- [2-(5-méthoxyindol-3-yl) éthyl] diacétamide (4)
N- [2-(2-acétyl-indol-3-yl) éthyl] acétamide (5)
N-[2-(1-acétyl-2-oxo-5-méthoxy-2,3-dihydroindol-3-yl)éthyl] acétamide (6)
N- [2-(5-méthoxyindol-3-yl) éthyl] glutarimide (7)
N-[2-(2-cyclohexylcarbonyl-5-méthoxyindol-3-yl) acétamide (8),
N-[2-(2-acétyl-5-méthoxyindol-3-yl) éthyl] diacétamide (9),
N-[2-(1-acétyl-5-méthoxyindol-3-yl)éthyl] diacétamide (10),
N-[2-(5-méthoxy-2-oxo-2, 3-dihydroindol-3-yl)éthyl] glutarimide (11)
N-[2-(6-acétyl-5-méthoxyindol-3-yl)éthyl] acétamide (12)
N-[2-(1-carbéthoxy-2-acétyl-5-méthoxyindol-3-yl)éthyl] acétamide (14)
N-[2-(2-acétyl-6-éthyl-5-méthoxyindol-3-yl) éthyl] acétamide (15)
N-[2-(1-acétyl-5-méthoxyindolin-3-yl) éthyl]acétamide (16)
N-[2-(1-acétyl-6-chloro-5-méthoxyindolin-3-yl)éthyl]acétamide (17)
N-[2-(1-benzyloxycarbonyl-5-méthoxyindolin-3-yl)éthyl]diacétamide (18)
N-[2-(1-trifluorométhanesulfonyl-5-méthoxyindolin-3-yl)éthyl]diacétamide (19)
N-[2-(6-(1-acétyl-3-(N,N-diacétyl-2-aminoéthyl)-5-méthoxyindolin-2-yl)-5méthoxyindol-3-yl)éthyl]diacétamide (20)
N-[2-(1-carbéthoxy-2-acétyl-5-méthoxyindolin-3-yl)éthyl]acétamide (22).

8. Dérivés selon l'une des revendications 1 à 6 pour leur utilisation en thérapie à titre de médicament.

9. Composition pharmaceutique **caractérisée en ce qu'**elle comprend un dérivé selon l'une des revendications 1 à 7 et au moins un excipient acceptable pharmaceutiquement.

10. Composition cosmétique **caractérisée en ce qu'**elle comprend un dérivé selon l'une des revendications 1 à 7.

## Patentansprüche

1. Derivate, die ausgewählt sind aus der Gruppe bestehend aus:
- den Derivaten der allgemeinen Formel I 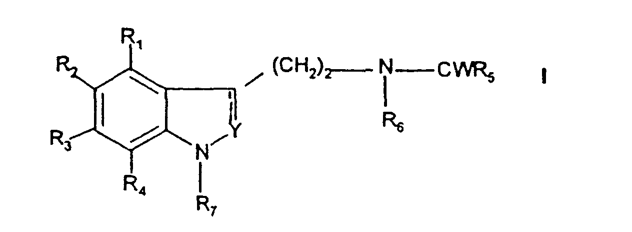 in der
W ein Sauerstoff- oder Schwefelatom oder einen Rest =NR₁₂ darstellt, wobei R₁₂ ein Wasserstoffatom, einen (C₁-C₆)-Alkyl-, Aryl-, Aralkylrest mit einem (C₁-C₆)-Alkylteil, (C₃-C₆)-Cycloalkylrest darstellt,
Y --- einen Rest der Formel CR₈=, CW₁- oder CHR₂₀ darstellt, wobei W₁ die gleiche Definition wie W oben aufweist,
R₁ bis R₄ unabhängig voneinander ein Wasserstoffatom, einen Hydroxylrest, einen (C₁-C₆)-Alkylrest, einen Alkoxyrest mit einem (C₁-C₆)-Alkylteil, einen Aryloxyrest, einen Aralkyloxyrest mit einem (C₁-C₆)-Alkylteil, ein Halogen oder Nitro darstellen,
R₅ ein Wasserstoffatom, einen (C₁-C₆)-Alkylrest, einen Arylrest, einen Aralkylrest mit einem (C₁-C₆)-Alkylteil, einen (C₁-C₆)-Perhalogenalkylrest, einen Aminorest, einen (C₁-C₆)-Alkylamino- oder (C₁-C₆)-Dialkylaminorest oder einen Alkoxyrest mit einem (C₁-C₆)-Alkylteil darstellt,
R₆ und R₂₀ unabhängig voneinander ein Wasserstoffatom, einen Hydroxylrest, einen (C₁-C₆)-Alkylrest, einen (C₃-C₆)-Cycloalkylrest, einen Alkoxyrest mit einem (C₁-C₆)-Alkylteil, einen Aryloxyrest, einen Aralkoxyrest mit einem (C₁-C₆)-Alkylteil, einen (C₁-C₆)-Alkylthio-, Arylthio-, Ar-(C₁-C₆)-alkylthio-, Halogen-, Nitrorest oder eine ungesättigte aliphatische Kette (C₂-C₆)-Alkenyl, (C₂-C₆)-Alkinyl, (C₁-C₆)-Alkyl, Aryl, Aralkyl mit einem (C₁-C₆)-Alkylteil, einen (C₁-C₆)-Perhalogenalkylrest, einen Amino-, (C₁-C₆)-Alkylamino-, (C₁-C₆)-Dialkylamino-, Arylamino-, Diarylamino-, Aralkylaminorest mit einem (C₁-C₆)-Alkylteil, einen Rest der Form CV-R₁₁ oder QCVR₁₁, in dem V ein Sauerstoff- oder Schwefelatom oder einen Iminrest =N-R₁₂ darstellt, R₁₁ eine der Bedeutungen von R₁ aufweist, darstellen,
worin der Ausdruck Aryl eine Phenyl-, Thienyl-, Furanyl-, Pyridyl- oder Naphthylgruppe darstellt, die gegebenenfalls durch einen oder mehrere Substituenten substituiert ist, die aus (C₁-C₆)-Alkyl-, Alkoxyresten mit einem (C₁-C₆)-Alkylteil oder Halogen ausgewählt sind,
Q ein Sauerstoff- oder Schwefelatom darstellt,
R₇ eine der Bedeutungen von R₁ aufweist, ausser dass es nicht Hydroxyl sein kann, oder einen Rest SO₂R₂₆ darstellen kann, in dem R₂₆ ein (C₁-C₆)-Alkylrest oder (C₁-C₆)-Halogenalkylrest, insbesondere CF₃, ist,
R₈ eine der Bedeutungen von R₁ aufweist und auch ein Halogenatom (Chlor, Brom, lod, Fluor), eine Gruppe Q'-CV'-R'₁₁ darstellen kann, in der Q'V' und R'₁₁ jeweils die gleichen sind wie QV und R₁₁, die oben definiert sind, oder
R₅ und R₆ -(CH₂)ₘ-CW' darstellen, wobei m eine ganze Zahl von 2 und 3 ist und W' die gleiche Bedeutung wie W aufweist, wobei das Carbonyl oder Thiocarbonyl -CW'- direkt mit dem Stickstoff verbunden ist und der Rest -(CH₂)ₘ seinerseits an das Carbonyl oder Thiocarbonyl der Gruppe -CW- gebunden ist,
mit der Bedingung, dass mindestens eines von R₁, R₆, R₇, R₈, R₂₀ einen (C₁-C₆)-Alkylcarbonyl- oder einen (C₁-C₆)-Alkylthiocarbonylrest darstellt,
- N-[2-(5-Methoxyindol-3-yl)ethyl]glutarimid (7)
- N-[2-(2-Cyclohexylcarbonyl-5-methoxyindol-3-yl)ethyl]acetamid (8)
- N-[2-(5-Methoxy-2-oxo-2,3-dihydroindol-3-yl)ethyl]glutarimid (11) und
- N-[2-(6-Acetyl-5-methoxyindol-3-yl)ethyl]acetamid (12),
deren Racemate, deren reine Enantiomere oder deren Mischungen in allen Verhältnissen und deren therapeutisch annehmbare Salze.

2. Derivate nach Anspruch 1, **dadurch gekennzeichnet, dass** mindestens einer der Substituenten R₂ oder R₃ von einem Wasserstoffatom verschieden ist und vorzugsweise einen Hydroxyl- oder Alkoxyrest mit einem (C₁-C₆)-Alkylteil, insbesondere einen Methoxyrest, darstellt.

3. Derivate nach einem der Ansprüche 1 oder 2, **dadurch gekennzeichnet, dass** R₁ und R₄ ein Wasserstoffatom darstellen.

4. Derivate nach den Ansprüchen 1 bis 3, **dadurch gekennzeichnet, dass** R₅ ein (C₁-C₆)-Alkylrest, vorzugsweise ein Methyl- oder ein Ethylrest, oder ein Perfluormethyl-, Perfluorethyl- oder Perfluorpropylrest, vorzugsweise ein Perfluorethylrest, ist.

5. Derivate nach einem der Ansprüche 1 bis 4, **dadurch gekennzeichnet, dass** R₇ einen (C₁-C₆)-Alkylcarbonylrest darstellt.

6. Derivate nach einem der Ansprüche 1 bis 5, **dadurch gekennzeichnet, dass** Y ---- einen zweiwertigen Rest der Formel CR₈= darstellt und R₈ ein Wasserstoffatom oder einen (C₁-C₆)-Alkylcarbonylrest darstellt.

7. Derivate nach Anspruch 1, die ausgewählt sind aus den folgenden Verbindungen:
N-[2-(1-Acetyl-5-methoxyindol-3-yl)ethyl]acetamid (1),
N-[2-(2-Acetyl-5-methoxyindol-3-yl)ethyl]acetamid (2),
N-[2-(2-Acetyl-5-methoxyindol-3-yl)ethyl]propionamid (3),
N-[2-(5-Methoxyindol-3-yl)ethyl]diacetamid (4),
N-[2-(2-Acetylindol-3-yl)ethyl]acetamid (5),
N-[2-(1-Acetyl-2-oxo-5-methoxy-2,3-dihydroindol-3-yl)ethyl]acetamid (6),
N-[2-(5-Methoxyindol-3-yl)ethyl]glutarimid (7),
N-[2-(2-Cyclohexylcarbonyl-5-methoxyindol-3-yl)ethyl]acetamid (8),
N-[2-(2-Acetyl-5-methoxyindol-3-yl)ethyl]diacetamid (9),
N-[2-(1-Acetyl-5-methoxyindol-3-yl)ethyl]diacetamid (10),
N-[2-(5-Methoxy-2-oxo-2,3-dihydroindol-3-yl)ethyl]glutarimid (11),
N-[2-(6-Acetyl-5-methoxyindol-3-yl)ethyl]acetamid (12),
N-[2-(1-Carbethoxy-2-acetyl-5-methoxyindol-3-yl)ethyl]acetamid (14),
N-[2-(2-Acetyl-6-ethyl-5-methoxyindol-3-yl)ethyl]acetamid (15),
N-[2-(1-Acetyl-5-methoxyindolin-3-yl)ethyl]acetamid (16),
N-[2-(1-Acetyl-6-chlor-5-methoxyindolin-3-yl)ethyl]acetamid (17),
N-[2-(1-Benzyloxycarbonyl-5-methoxyindolin-3-yl)ethyl]diacetamid (18),
N-[2-(1-Trifluormethansulfonyl-5-methoxyindolin-3-yl)ethyl]diacetamid (19),
N-[2-(6-(1-Acetyl-3-(N,N-diacetyl-2-aminoethyl)-5-methoxyindolin-2-yl)-5-methoxyindol-3-yl)ethyl]diacetamid (20),
N-[2-(1-Carbethoxy-2-acetyl-5-methoxyindolin-3-yl)ethyl]acetamid (22).

8. Derivate nach einem der Ansprüche 1 bis 6 für die therapeutische Verwendung als Medikament.

9. Pharmazeutische Zusammensetzung, **dadurch gekennzeichnet, dass** sie ein Derivat nach einem der Ansprüche 1 bis 7 und mindestens einen pharmazeutisch annehmbaren Träger umfasst.

10. Kosmetische Zusammensetzung, **dadurch gekennzeichnet, dass** sie ein Derivat nach einem der Ansprüche 1 bis 7 umfasst.

## Claims

1. Derivatives chosen from the group consisting of:
- the derivatives of general formula I 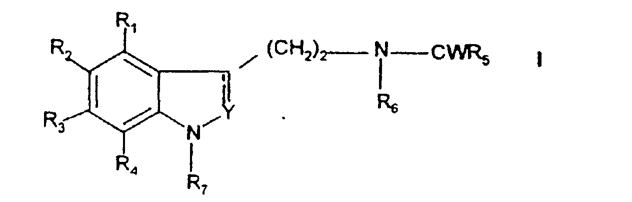 in which
**W** represents an oxygen or sulphur atom or a radical =NR₁₂, R₁₂ being a hydrogen atom, a C₁-C₆ alkyl radical, an aryl radical, an aralkyl radical whose alkyl residue is C₁-C₆, or a C₃-C₆ cycloalkyl radical,
**Y ---** represents a radical of formula CR₈=, CW₁- or CHR₂₀, W₁ having the same definition as W above,
**R**_{**1**} **to R**_{**4**} represent, independently of each other, a hydrogen atom, a hydroxyl radical, a C₁-C₆ alkyl radical, an alkoxy radical whose alkyl residue is C₁-C₆, an aryloxy radical, an aralkoxy radical whose alkyl residue is C₁-C₆, a halo radical or a nitro radical,
**R**_{**5**} represents a hydrogen atom, a C₁-C₆ alkyl radical, an aryl radical, an aralkyl radical whose alkyl residue is C₁-C₆, a C₁-C₆ perhalo alkyl radical, an amino radical, a C₁-C₆ alkylamino or C₁-C₆ dialkylamino radical, or an alkoxy radical whose alkyl residue is C₁-C₆,
**R**_{**6**} **and R**_{**20**} represent, independently of each other, a hydrogen atom, a hydroxyl radical, a C₁-C₆ alkyl radical, a C₃-C₆ cycloalkyl radical, an alkoxy radical whose alkyl residue is C₁-C₆, an aryloxy radical, an aralkoxy radical whose alkyl residue is C₁-C₆, a C₁-C₆ alkylthio radical, an arylthio radical, a C₁-C₆ aralkylthio radical, a halo radical, a nitro radical or an unsaturated aliphatic chain, a C₂-C₆ alkenyl radical, a C₂-C₆ alkynyl radical, a C₁-C₆ alkyl radical, an aryl radical, an aralkyl radical whose alkyl residue is C₁-C₆, a C₁-C₆ perhalo alkyl radical, an amino radical, a C₁-C₆ alkylamino radical, a C₁-C₆ dialkylamino radical, an arylamino radical, a diarylamino radical, an aralkylamino radical whose alkyl residue is C₁-C₆, a radical of the form CV-R₁₁ or QCVR₁₁ in which V represents an oxygen or sulphur atom or an imine radical =N-R₁₂, R₁₁ has one of the meanings of R₁,
in which the term aryl represents a phenyl, thienyl, furanyl, pyridyl or naphthyl group, optionally substituted with one or more substituents chosen from a C₁-C₆ alkyl radical, an alkoxy radical whose alkyl residue is C₁-C₆ or a halo radical,
**Q** represents an oxygen or sulphur atom,
**R**_{**7**} has one of the meanings of R₁, except that it cannot be hydroxyl, or it can represent a radical SO₂R₂₆, R₂₆ being a C₁-C₆ alkyl radical or a C₁-C₆ halo alkyl, in particular CF₃, radical,
**R**_{**8**} has one of the meanings of R₁ and may also represent a halogen atom (chlorine, bromine, iodine, fluorine), a group Q'-CV'-R'₁₁ in which Q', V' and R'₁₁ are respectively like Q, V and R₁₁ defined above, or
**R**_{**5**} **and R**_{**6**} represent -(CH₂)ₘ-CW'-, m being an integer between 2 and 3 and W' having the same meaning as W, the carbonyl or thiocarbonyl-CW'- being directly linked to nitrogen and the radical -(CH₂)ₘ being, for its part, linked to the carbonyl or thiocarbonyl of the -CW- group,
provided that at least one of R₁, R₆, R₇, R₈, R₂₀ represents a C₁-C₆ alkylcarbonyl or C₁-C₆ alkylthiocarbonyl radical,
- N-[2-(5-methoxyindol-3-yl)ethyl]glutarimide (7),
- N-[2-(2-cyclohexylcarbonyl-5-methoxyindol-3-yl)ethyl]acetamide (8)
- N-[2-(5-methoxy-2-oxo-2,3-dihydroindol-3-yl)ethyl]glutarimide (11) and
- N-[2-(6-acetyl-5-methoxyindol-3-yl)ethyl]acetamide (12),
their racemates, their pure enantiomers, or their mixtures in any proportions, and their therapeutically acceptable salts.

2. Derivatives according to Claim 1, **characterized in that** at least one of the substituents R₂ or R₃ is different from a hydrogen atom and preferably represents a hydroxyl or alkoxy radical whose alkyl residue is C₁-C₆, in particular a methoxy radical.

3. Derivatives according to either of Claims 1 and 2, **characterized in that** R₁ and R₄ represent a hydrogen atom.

4. Derivatives according to Claims 1 to 3, **characterized in that** R₅ is a C₁-C₆ alkyl radical, preferably a methyl or an ethyl, or a perfluoromethyl, perfluoroethyl or perfluoropropyl radical, preferably a perfluoroethyl radical.

5. Derivatives according to one of Claims 1 to 4, **characterized in that** R₇ represents a C₁-C₆ alkylcarbonyl radical.

6. Derivative according to one of Claims 1 to 5, **characterized in that** Y--- represents a divalent radical of formula CR₈= and R₈ represents a hydrogen atom or a C₁-C₆ alkylcarbonyl radical.

7. Derivatives according to Claim 1, selected from the following compounds:
N-[2-(1-acetyl-5-methoxyindol-3-yl)ethyl]acetamide (1),
N-[2-(2-acetyl-5-methoxyindol-3-yl)ethyl]acetamide (2),
N-[2-(2-acetyl-5-methoxyindol-3-yl)ethyl]-propionamide (3),
N-[2-(5-methoxyindol-3-yl)ethyl]diacetamide (4),
N-[2-(2-acetylindol-3-yl)ethyl]acetamide (5),
N-[[2-(1-acetyl-2-oxo-5-methoxy-2,3-dihydroindol-3-yl)ethyl]acetamide (6),
N-[2-(5-methoxyindol-3-yl)ethyl]glutarimide (7),
N-[2-(2-cyclohexylcarbonyl-5-methoxyindol-3-yl ethyl) acetamide (8),
N-[2-(2-acetyl-5-methoxyindol-3-yl)ethyl]-diacetamide (9),
N-[2-(1-acetyl-5-methoxyindol-3-yl)ethyl]-diacetamide (10),
N-[2-(5-methoxy-2-oxo-2,3-dihydroindol-3-yl)-ethyl]glutarimide (11),
N-[2-(6-acetyl-5-methoxyindol-3-yl)ethyl]acetamide (12),
N-[2-(1-carbethoxy-2-acetyl-5-methoxyindol-3-yl)ethyl]acetamide (14),
N-[2-(2-acetyl-6-ethyl-5-methoxyindol-3-yl)ethyl]acetamide (15),
N-[2-(1-acetyl-5-methoxyindolin-3-yl)ethyl]-acetamide (16),
N-[2-(1-acetyl-6-chloro-5-methoxyindolin-3-yl)ethyl]acetamide (17),
N-[2-(1-benzyloxycarbonyl-5-methoxyindolin-3-yl)ethyl]diacetamide (18),
N-[2-(1-trifluoromethanesulphonyl-5-methoxyindolin-3-yl)ethyl]diacetamide (19),
N-[(2-(6-(1-acetyl-3-(N,N-diacetyl-2-aminoethyl)-5-methoxyindolin-2-yl)-5-methoxyindol-3-yl)ethyl]diacetamide (20),
N-[2-(1-carbethoxy-2-acetyl-5-methoxyindolin-3-yl)ethyl]acetamide (22).

8. Derivatives according to one of Claims 1 to 6, for their use in therapy as a medicament.

9. Pharmaceutical composition, **characterized in that** it comprises a derivative according to one of Claims 1 to 7 and at least one pharmaceutically acceptable excipient.

10. Cosmetic composition, **characterized in that** it comprises a derivative according to one of Claims 1 to 7.
